# EUROPÄISCHE PATENTSCHRIFT

(11) **EP 0 555 236 B1**
(45) Veröffentlichungstag und Bekanntmachung des Hinweises auf die Patenterteilung: **13.09.1995**
(21) Anmeldenummer: 91917353.4
(22) Anmeldetag: 10.10.1991
(51) Int. Cl.: G01N 27/22, G01N 33/28, G01N 27/07

(54) **Sensor zur Messung des Mischungsverhältnisses von Flüssigkeiten**
Sensor for measuring the mixing ratio of fluids
Capteur pour la mesure du rapport de mélange de liquides

(30) Priorität: 30.10.1990 DE 4034471
(43) Veröffentlichungstag der Anmeldung: 18.08.1993
(73) Patentinhaber: ROBERT BOSCH GMBH, 70442 Stuttgart (DE)
(72) Erfinder: SEIPLER, Dieter, D-7410 Reutlingen (DE); ZIEGENBEIN, Botho, D-7410 Reutlingen (DE); MAIHOEFER, Bernd, D-7410 Reutlingen (DE)
(86) Internationale Anmeldenummer: DE9100798
(87) Internationale Veröffentlichungsnummer: WO9208126

(56) Entgegenhaltungen:
- EP-A- 0 280 229
- EP-A- 0 291 363
- DE-A- 3 843 097
- PATENT ABSTRACTS OF JAPAN vol. 6, no. 133 (P-129)(1011) 20. Juli 1982 & JP-A-57 057 253
- PATENT ABSTRACTS OF JAPAN vol. 12, no. 290 (P-742) 9. August 1988 & JP-A-63 067 556

## Beschreibung

### Stand der Technik

Die Erfindung geht aus von einem Sensor zur Messung des Mischungsverhältnisses von Flüssigkeiten nach der Gattung des Hauptanspruchs.

In der europäischen Patentanmeldung 0 335 168 A2 wird ein Sensor beschrieben, der den Alkoholgehalt eines Kraftstoffgemisches zum optimalen Betrieb einer Brennkraftmaschine bestimmt. Die Meßzelle des Sensors weist einen Zufluß und einen Abfluß auf, so daß das Kraftstoffgemisch die Meßzelle durchströmen kann. Die Außenwände der Meßzelle und ein Mittelzylinder sind zumindest teilweise aus leitendem Material und bilden einen Zylinderkondensator, der Bestandteil einer Auswerteschaltung ist. Die Kapazität des Zylinderkondensators wird durch die Zusammensetzung des das Dielektrikum bildenden Kraftstoffgemisches beeinflußt. Mit der Zusammensetzung ändert sich neben der Kapazität auch der Leitwert des Kraftstoffgemischs, der ebenfalls über die Elektroden des Kondensators erfaßt wird. Diese Anordnung wurde bislang in makroskopischen Dimensionen mit den dabei üblichen Fertigungsmethoden realisiert.

### Vorteile der Erfindung

Der erfindungsgemäße Sensor mit den kennzeichnenden Merkmalen des Hauptanspruchs hat demgegenüber den Vorteil, daß er sich in sehr kleinen Ausmaßen realisieren läßt, so daß sein Einsatz keinen nennenswerten räumlichen Mehraufwand darstellt. Vorteilhaft ist außerdem, daß das Keramikgehäuse der Meßzelle eine sehr gute Medienverträglichkeit, insbesondere für Kraftstoffgemische, aufweist. Von Vorteil ist ferner, daß sich der Sensor mit bekannten und gut beherrschbaren Methoden und Materialien der Dickschichttechnik aufbauen läßt. Dabei kann die Gehäusefertigung und das Aufbringen von Schaltungselementen, wie den Elektroden des Meßkondensators, in einem Verfahrensschritt erfolgen.

Durch die in den Unteransprüchen aufgeführten Maßnahmen sind vorteilhafte Weiterbildungen des im Hauptanspruch angegebenen Sensors möglich. Es ist besonders vorteilhaft, wenn die Meßzelle einen weiteren abgeschlossenen Hohlraum aufweist, auf dessen Innenwänden Elektroden aufgebracht sind, die zusammen mit einem den weiteren Hohlraum erfüllenden Referenzmedium einen Referenzkondensator bilden, der ebenfalls Bestandteil der Auswerteschaltung ist. Durch das Einbeziehen eines Referenzkondensators in den Meßvorgang lassen sich Langzeitdriften und Temperaturgänge des Sensors reduzieren, da sich die Kennlinie des Referenzkondensators bei gleichen Betriebsbedingungen in ähnlicher Weise ändert wie die Kennlinie des ersten Kondensators, dessen Dielektrikum die zu messende Flüssigkeit ist.

Da das Referenzmedium in den weiteren Hohlraum eingeschlossen sein soll aber thermischer Ausdehnung unterliegt, ist es vorteilhaft, den weiteren Hohlraum über einen Durchlaß mit einem Ausgleichsvolumen zu verbinden. Eine vorteilhafte Ausgestaltung des Ausgleichsvolumens, das ebenfalls mit dem Referenzmedium erfüllt ist, besteht darin, eine Begrenzungswand des Ausgleichsvolumens als elastische Keramikmembran auszubilden. Ein weiteres vorteilhaftes Verfahren der Kompensation von Temperaturgängen des Sensors besteht darin, die Temperatur der Flüssigkeit oder des Referenzmediums direkt zu messen. Dies kann vorteilhaft durch temperaturabhängige Widerstände erfolgen, die in Dickschichttechnik auf die Innenwände eines Hohlraums oder des Ausgleichsvolumens aufgebracht sind und so in unmittelbarem Kontakt mit der Flüssigkeit oder dem Referenzmedium stehen. Der Aufbau der Meßzelle in Dickschichttechnik ermöglicht das Aufbringen von Teilen oder der gesamten Auswerteschaltung in Dickschicht-Hybridtechnik auf der Meßzelle selbst, was die Störsicherheit der Signalauswertung erhöht. Beim Aufbau der Meßzelle lassen sich ferner kostengünstige Keramikformteile verwenden, in denen schon der Zufluß und der Abfluß der Meßzelle ausgebildet sind. Die Hohlräume der Meßzelle werden durch das Fehlen der Verbindungsschicht in bestimmten Bereichen gebildet. Besonders geeignet als Verbindungsschicht ist in diesem Zusammenhang eine Glasschicht, die sich in Form einer Dickschichtpaste, einfach strukturiert auf einen Keramikträger aufbringen läßt und durch die beim Verglasen die Verbindung zu einem weiteren Keramikträger hergestellt werden kann. Dieses Verfahren ist hinreichend bekannt und ausgereift.

### Zeichnung

Zwei Ausführungsbeispiele der Erfindung sind in der Zeichnung dargestellt und in der nachfolgenden Beschreibung näher erläutert.

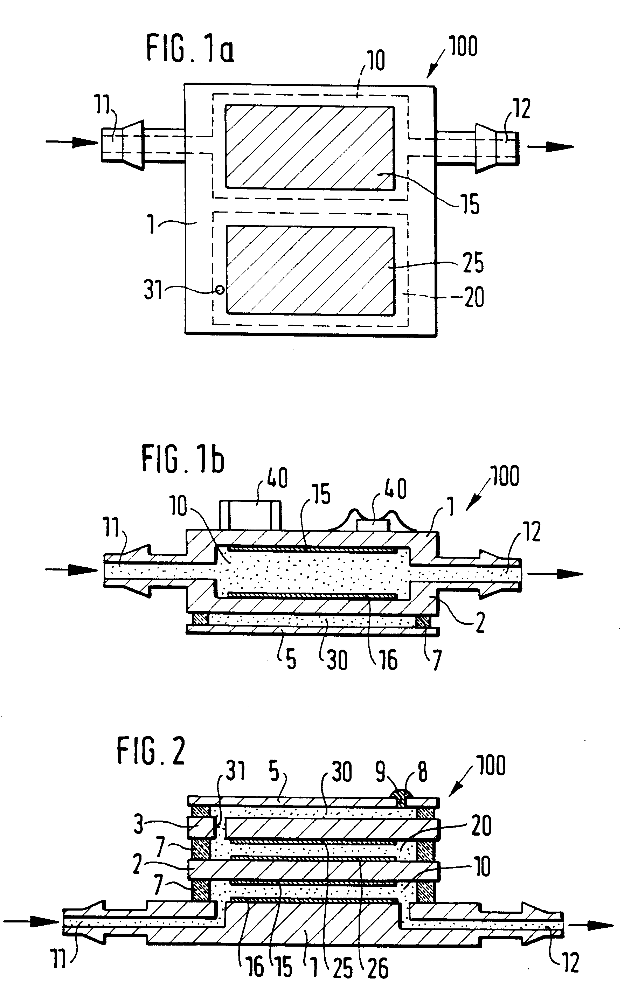

Es zeigen Figur 1a die Aufsicht auf einen Sensor, Figur 1b den Querschnitt des in 1a dargestellten Sensors und in Figur 2 den Querschnitt eines weiteren Sensors.

### Beschreibung des Ausführungsbeispiels

In den Figuren 1a und b ist mit 100 eine Meßzelle bezeichnet, die aus als Keramikformteile ausgebildeten Keramikträgern 1 und 2 gefertigt ist. Das Keramikformteil 1 bildet eine obere Abdeckung, in der auch schon die Oberseiten eines Zuflusses 11 und eines Abflusses 12 ausgebildet sind: das Keramikformteil 2 bildet das als Unterseite der Meßzelle dienende Gegenstück. Die beiden Keramikformteile 1 und 2 sind über eine Verbindungsschicht, die in den in Figur 1 dargestellten Ansichten nicht zu sehen ist, miteinander verbunden. Im Innern der Meßzelle 100 befinden sich zwei voneinander getrennte Hohlräume 10 und 20, die durch bereichsweises Fehlen der Verbindungsschicht gebildet sind. Eine Flüssigkeit, deren Mischungsverhältnis bestimmt werden soll, kann über den Zufluß 11 und den Abfluß 12 durch die Meßzelle 100 geleitet werden, wobei nur der eine Hohlraum 10 durchströmt wird. Die Strömungsrichtung ist durch die Pfeile angedeutet. Auf zwei gegenüberliegenden, von den Keramikträgern 1, 2 gebildeten Innenwänden des Hohlraums 10 sind jeweils eine Elektrode 15 und 16 aufgebracht. Wie in Figur 1b dargestellt, bilden die beiden Elektroden 15 und 16 einen Plattenkondensator mit der durchströmenden Flüssigkeit als Dielektrikum, dessen Kapazität sich je nach Mischungsverhältnis der Flüssigkeit ändert.

Dieser Meßkondensator ist Teil einer Auswerteschaltung, die in Figur 1b mit 40 bezeichnet ist, und teilweise direkt in Dickschicht-Hybridtechnik auf die Meßzelle aufgebracht ist. Auf zwei gegenüberliegenden Innenwänden eines weiteren Hohlraums 20 sind wie im ersten Hohlraum 10 zwei Elektroden aufgebracht, von denen nur eine Elektrode 25 in Figur 1a dargestellt ist, da der zweite Hohlraum 20 neben dem ersten Hohlraum 10 angeordnet ist, so daß die Begrenzungswände des ersten Hohlraums 10 und des zweiten Hohlraums 20 von den selben Keramikträgern 1 und 2 gebildet werden. In dem zweiten Hohlraum 20 befindet sich ein Referenzmedium, das als Dielektrikum des durch die Elektroden im zweiten Hohlraum 20 gebildeten Referenzkondensators dient, der ebenfalls Bestandteil der Auswerteschaltung 40 ist. Durch diesen Referenzkondensator lassen sich zum einen Temperaturgänge des Sensors kompensieren, zum anderen Langzeitdriften der Kennlinie erfassen, da die Betriebsbedingungen des Meß- und des Referenzkondensators weitgehend übereinstimmen und deshalb zu erwarten ist, daß sich ihre Kennlinien in gleicher Weise mit der Zeit verändern. In Figur 1a ist mit 31 ein Durchlaß bezeichnet, dieser Durchlaß 31 besteht in einer Bohrung des Keramikträgers 2 im Bereich des Hohlraums 20. Er stellt eine Verbindung zwischen dem Hohlraum 20 und einem Ausgleichsvolumen 30 her, das ebenfalls mit dem Referenzmedium gefüllt ist. Das Ausgleichsvolumen 30 ist ein Hohlraum zwischen dem Keramikträger 2 und einer über eine Zwischenschicht 7 auf den Keramikträger 2 aufgebrachten, elastischen Keramikmembran 5. Diese Keramikmembran 5 ermöglicht eine beispielsweise temperaturbedingte Volumenänderung des Referenzmediums, das den zweiten Hohlraum 20 und das Ausgleichsvolumen 30 vollständig erfüllt.

Schaltungselemente, wie Widerstände und Elektroden von Kondensatoren, lassen sich sehr einfach in Siebdrucktechnik auf das Keramiksubstrat der Meßzelle 100 aufdrucken. Dazu werden Stahl- oder Polyester-Siebe so präpariert, daß ihre Maschen für die zu bedruckenden Stellen offen und dazwischen geschlossen sind. Mit einer Druckrakel wird eine entsprechende Paste durch das Sieb auf das Keramiksubstrat gestrichen. Dabei können auch mehrere Druckschritte erforderlich sein, bei denen unterschiedliche Pasten verwendet werden. Nach dem Druck wird die Paste bei etwas erhöhter Temperatur getrocknet und anschließend gebrannt. Mit dieser Methode werden auch die Keramikträger verbunden. Als Verbindungsschicht wird vorzugsweise eine strukturierte Glasschicht verwendet. Vorteilhaft ist es auch, neben den Elektroden im Innern der Hohlräume 10 und 20 oder auch im Ausgleichsvolumen 30 temperaturabhängige Widerstände aufzudrucken und damit die Temperatur der Flüssigkeit und/oder des Referenzmediums zu erfassen.

In Figur 2 ist eine Meßzelle 100 dargestellt, bei der zwei Hohlräume 10 und 20 übereinander angeordnet sind. Die Meßzelle 100 in diesem Beispiel ist durch Aufeinanderglasen von drei Keramikträgern 1, 2 und 3 und einer Keramikmembran 5 entstanden. Der Keramikträger 1 ist ein Keramikformteil, in dem ein Zufluß 11 und ein Abfluß 12 ausgebildet sind. Der Hohlraum 10 ist als Zwischenraum zwischen den Keramikträgern 1 und 2 ausgebildet. Im Bereich des Hohlraums 10 sind auf den Keramikträgern 1 und 2 Elektroden 15 und 16 aufgebracht. Der Hohlraum 10 steht ferner in Verbindung mit den in dem Keramikträger 1 ausgebildeten Zufluß 11 und Abfluß 12, so daß die Flüssigkeit, deren Mischungsverhältnis bestimmt werden soll, das Dielektrikum des im Hohlraum 10 angeordneten Kondensators bildet. Ein Referenzmedium ist in dem zweiten Hohlraum 20 zwischen den Keramikträgern 2 und 3 eingeschlossen, in dem ebenfalls zwei Elektroden 25 und 26 einen Referenzkondensator bilden. Der Hohlraum 20 steht über eine Bohrung 31 mit einem Ausgleichsvolumen 30 in Verbindung, das durch eine elastische Keramikmembran 5 begrenzt wird. Der Hohlraum 20 und das Ausgleichsvolumen 30 sind in der selben Weise gefertigt wie der Hohlraum 10 mit Hilfe von Verbindungsschichten 7. Die Seitenwände werden jeweils von den strukturierten Glasverbindungsschichten 7 gebildet. In der Keramikmembran 5 befindet sich eine Bohrung 9, über die das Referenzmedium in dem Hohlraum 20 und das Ausgleichsvolumen 30 eingefüllt ist. Als Verschluß 8 für die Bohrung 9 kann vorteilhaft ein Lottropfen verwendet werden, der auf die metallisierte Bohrung 9 aufgebracht wird. In Figur 2 wurde auf die Darstellung einer Auswerteschaltung verzichtet. Die Funktionsweise des in Figur 2 dargestellten Sensors entspricht der des in Figur 1 dargestellten Sensors.

## Patentansprüche

1. Sensor zur Messung des Mischungsverhältnisses von Flüssigkeiten, insbesondere von Kraftstoffgemischen, mit einer Meßzelle, die einen Hohlraum mit einem Zufluß und einem Abfluß aufweist, durch den die Flüssigkeit geleitet wird, wobei Bereiche der Wandung des Hohlraums elektrisch leitend sind und mit mindestens einer weiteren elektrisch leitenden Fläche einen Kondensator bilden, der Bestandteil einer Auswerteschaltung ist, dadurch gekennzeichnet, daß die Meßzelle (100) aus mindestens zwei Keramikträgern (1, 2) gefertigt ist, daß die mindestens zwei Keramikträger (1, 2) über mindestens eine Verbindungsschicht (7) in einer Schichtanordnung verbunden sind, daß der Hohlraum (10) durch bereichsweises Fehlen der mindestens einen Verbindungsschicht (7) zwischen zwei Keramikträgern (1, 2) erzeugt ist und daß im Bereich des Hohlraums (10) jeweils mindestens eine Elektrode (15, 16) in Dickschichttechnik auf die den Hohlraum (10) begrenzenden Keramikträger (1, 2) aufgebracht ist.

2. Sensor nach Anspruch 1, dadurch gekennzeichnet, daß die Meßzelle (100) mindestens einen weiteren Hohlraum (20) aufweist, der durch bereichsweises Fehlen der mindestens einen Verbindungsschicht (7) zwischen zwei Keramikträgern (1, 2, 3) erzeugt ist, daß im Bereich des weiteren Hohlraums (20) jeweils mindestens eine Elektrode (25, 26) in Dickschichttechnik auf die den weiteren Hohlraum (20) begrenzenden Keramikträger (1, 2 , 3) aufgebracht ist, daß der weitere Hohlraum (20) mit einem Referenzmedium gefüllt ist und daß der aus den Elektroden (25, 26) und dem Referenzmedium gebildeten Kondensator Bestandteil der Auswerteschaltung (40) ist.

3. Sensor nach Anspruch 2, dadurch gekennzeichnet, daß der weitere Hohlraum (20) über einen Durchlaß (31) mit einem dritten Hohlraum (30) als Ausgleichsvolumen in Verbindung steht.

4. Sensor nach Anspruch 3, dadurch gekennzeichnet, daß der dritte Hohlraum (30) zumindest teilweise von einer elastischen Keramikmembran (5) begrenzt wird.

5. Sensor nach einem der vorhergehenden Ansprüche, dadurch gekennzeichnet, daß in mindestens einem Hohlraum (10, 20, 30) Mittel zur Erfassung der Temperatur der Flüssigkeit und/oder des Referenzmediums, vorzugsweise mindestens ein temperaturabhängiger, in Dickschichttechnik aufgebrachter Widerstand, vorhanden sind.

6. Sensor nach einem der vorhergehenden Ansprüche, dadurch gekennzeichnet, daß Teile der Auswerteschaltung (40) in Dickschicht-Hybridtechnik auf die Meßzelle (100) aufgebracht sind.

7. Sensor nach einem der vorhergehenden Ansprüche, dadurch gekennzeichnet, daß die mindestens eine Verbindungsschicht (7) eine in Dickschichttechnik strukturierte Glasschicht ist.

8. Sensor nach einem der vorhergehenden Ansprüche, dadurch gekennzeichnet, daß die Keramikträger (1, 2, 3) Formkeramikteile sind, in denen der Zufluß (11) und der Abfluß (12) ausgebildet sind.

9. Sensor nach einem der Ansprüche 2 - 8, dadurch gekennzeichnet, daß das Referenzmedium über eine Bohrung (9) in der Meßzelle (100) in den weiteren Hohlraum (20) bzw. dritten Hohlraum (30) eingebracht ist und daß die Bohrung (9) verschlossen ist, so daß das Referenzmedium in dem weiteren Hohlraum (20) und dem dritten Hohlraum (30) eingeschlossen ist.

## Claims

1. Sensor for measuring the mixing ratio of fluids, especially fuel mixtures, having a measurement cell which has a hollow space with an inflow and an outflow through which the fluid is directed, regions of the wall of the hollow space being electrically conductive and forming, with at least one further electrically conductive surface, a capacitor which is a component of an evaluation circuit, characterized in that the measurement cell (100) is produced from at least two ceramic carriers (1, 2), in that the at least two ceramic carriers (1, 2) are connected via at least one connecting layer (7) in a layer arrangement, in that the hollow space (10) is produced by the lack, in certain areas, of the at least one connecting layer (7) between two ceramic carriers (1, 2), and in that in each case at least one electrode (15, 16) produced using thick-film technology is provided in the region of the hollow space (10) on the ceramic carriers (1, 2) which bound the hollow space (10).

2. Sensor according to Claim 1, characterized in that the measurement cell (100) has at least one further hollow space (20) which is produced by the lack, in certain areas, of the at least one connecting layer (7) between two ceramic carriers (1, 2, 3), in that in each case at least one electrode (25, 26) produced using thick-film technology is provided in the region of the further hollow space (20) on the ceramic carriers (1, 2, 3) which bound the further hollow space (20), in that the further hollow space (20) is filled with a reference medium, and in that the capacitor which is formed from the electrodes (25, 26) and the reference medium is a component of the evaluation circuit (40).

3. Sensor according to Claim 2, characterized in that the further hollow space (20) is connected via an opening (31) to a third hollow space (30) as a compensation volume.

4. Sensor according to Claim 3, characterized in that the third hollow space (30) is bounded at least partially by an elastic ceramic diaphragm (5).

5. Sensor according to one of the preceding claims, characterized in that means for detecting the temperature of the fluid and/or of the reference medium, preferably at least one temperature-dependent resistor which is provided using thick-film technology are present in at least one hollow space (10, 20, 30).

6. Sensor according to one of the preceding claims, characterized in that parts of the evaluation circuit (40) are provided on the measurement cell (100) using thick-film hybrid technology.

7. Sensor according to one of the preceding claims, characterized in that the at least one connecting layer (7) is a glass layer which is structured using thick-film technology.

8. Sensor according to one of the preceding claims, characterized in that the ceramic carriers (1, 2, 3) are shaped ceramic parts in which the inflow (11) and the outflow (12) are constructed.

9. Sensor according to one of Claims 2 - 8, characterized in that the reference medium is filled into the further hollow space (20) or third hollow space (30) via a hole (9) in the measurement cell (100), and in that the hole (9) is closed off so that the reference medium is enclosed in the further hollow space (20) and the third hollow space (30).

## Revendications

1. Capteur pour mesurer le rapport de mélange de liquides, notamment de mélange de carburant, à l'aide d'une cellule de mesure présentant une cavité avec une entrée et une sortie pour le liquide, des zones de la paroi de la cavité étant conductrices électriques et formant avec au moins une autre surface conductrice électrique, un condensateur faisant partie d'un circuit d'exploitation, caractérisé en ce que la cellule de mesure (100) est réalisée en au moins deux supports en céramique (1, 2) qui sont reliés par au moins une couche de liaison (7) pour former une disposition à couches, la cavité (10) étant réalisée par la suppression par endroits d'au moins une couche de liaison (7) entre deux supports en céramique (1, 2) et en ce qu'au niveau de la cavité (10) il y a chaque fois au moins une électrode (15, 16) réalisée en technique des couches épaisses sur le support en céramique (1, 2) qui délimite la cavité (10).

2. Capteur selon la revendication 1, caractérisé en ce que la cellule de mesure (100) comporte au moins une autre cavité (20) réalisée par la suppression par endroits d'au moins une couche de liaison (7) entre deux supports en céramique (1, 2, 3) et au niveau de l'autre cavité (20) on a réalisé chaque fois au moins une électrode (25, 26) en technique des couches épaisses sur le support en céramique (1, 2, 3) qui délimite cette autre cavité (20), et cette cavité (20) est remplie d'un milieu de référence et le condensateur formé par les électrodes (25, 26) et le milieu de référence fait partie du circuit d'exploitation (40).

3. Capteur selon la revendication 2, caractérisé en ce que l'autre cavité (20) est reliée par un passage (31) à une troisième cavité (30) constituant un volume de compensation.

4. Capteur selon la revendication 3, caractérisé en ce que la troisième cavité (30) est délimitée au moins partiellement par une membrane en céramique élastique (5).

5. Capteur selon l'une des revendications précédentes, caractérisé en ce qu'au moins une cavité (10, 20, 30) comporte des moyens pour détecter la température du liquide et/ou du milieu de référence, de préférence au moins une résistance réalisée en technique des couches épaisses et dépendant de la température.

6. Capteur selon l'une des revendications précédentes, caractérisé en ce que les parties du circuit d'exploitation (40) sont appliquées sur la cellule de mesure (100) selon la technique hybride des couches épaisses.

7. Capteur selon l'une des revendications précédentes, caractérisé en ce qu'au moins une couche de liaison (7) est une couche de verre structurée en technique des couches épaisses.

8. Capteur selon l'une des revendications précédentes, caractérisé en ce que les supports en céramique (1, 2, 3) sont des pièces en céramique moulées dans lesquelles sont réalisées l'entrée (11) et la sortie (12). 9°) Capteur selon l'une des revendications 2 à 8, caractérisé en ce que le milieu de référence est introduit à travers un perçage (9) dans la cellule de mesure (100), dans l'autre cavité (20) ou dans la troisième cavité (30) et le perçage (9) est fermé pour que le milieu de référence soit emprisonné dans l'autre cavité (20) et dans la troisième cavité (30).
